(19) Europäisches Patentamt / European Patent Office / Office européen des brevets

(11) **EP 3 456 336 B1**

(12) **EUROPEAN PATENT SPECIFICATION**

(45) Date of publication and mention
of the grant of the patent:
**21.07.2021 Bulletin 2021/29**

(21) Application number: **17795603.4**

(22) Date of filing: **12.05.2017**

(51) Int Cl.:
**A61K 36/489** (2006.01)     **A61K 36/23** (2006.01)
**A61P 19/06** (2006.01)

(86) International application number:
**PCT/CN2017/084073**

(87) International publication number:
**WO 2017/193985 (16.11.2017 Gazette 2017/46)**

(54) **A CELERY SEED AND SOPHORA FLOWER BUD EXTRACT AND MEDICAL USE THEREOF**

ZUSAMMENGESETZTES SELLERIESAMEN- UND SOPHORABLÜTENKNOSPENEXTRAK UND
MEDIZINISCHE VERWENDUNG DAVON

EXTRAIT COMPOSÉ DE GRAINE DE CÉLERI ET DE BOURGEON DE FLEUR DE SOPHORA ET
LEUR UTILISATION MÉDICALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.05.2016 CN 201610313303**

(43) Date of publication of application:
**20.03.2019 Bulletin 2019/12**

(73) Proprietor: **Beijing Tianheng Junwei
Pharmaceutical
Tech Co., Ltd
Beijing 102600 (CN)**

(72) Inventors:
• **GAO, Yongliang**
  **Daxing District Beijing 102600 (CN)**
• **ZHANG, Guimin**
  **Daxing District Beijing 102600 (CN)**
• **JIANG, Qingwei**
  **Daxing District Beijing 102600 (CN)**
• **ZHANG, Qiang**
  **Daxing District Beijing 102600 (CN)**
• **QIN, Hua**
  **Daxing District Beijing 102600 (CN)**
• **DI, Yuan**
  **Daxing District Beijing 102600 (CN)**
• **YI, Weifeng**
  **Daxing District Beijing 102600 (CN)**
• **LIU, Baochuan**
  **Daxing District Beijing 102600 (CN)**
• **CHEN, Changhong**
  **Daxing District Beijing 102600 (CN)**
• **JIA, Hongqian**
  **Daxing District Beijing 102600 (CN)**
• **ZHANG, Tiejun**
  **Daxing District Beijing 102600 (CN)**
• **YANG, Wenbin**
  **Daxing District Beijing 102600 (CN)**
• **CUI, Jiangtao**
  **Daxing District Beijing 102600 (CN)**
• **LI, Bo**
  **Daxing District Beijing 102600 (CN)**
• **WU, Huangyan**
  **Daxing District Beijing 102600 (CN)**
• **CHEN, Haisheng**
  **Daxing District Beijing 102600 (CN)**
• **SUN, Jianning**
  **Daxing District Beijing 102600 (CN)**

(74) Representative: **Lahrtz, Fritz et al
Patentanwälte
Isenbruck Bösl Hörschler PartG mbB
Prinzregentenstraße 68
81675 München (DE)**

(56) References cited:
**CN-A- 101 181 345      CN-A- 102 008 525
CN-A- 102 048 775      CN-A- 103 947 948
CN-A- 105 535 048**

- CHEN D ET AL: "Traditional Chinese medicine composition, e.g. for promoting healing of wound comprises pseudoginseng, Salvia miltiorrhiza, Asiatic pennywort herb, and sophora flower bud", WPI / THOMSON,, vol. 2010, no. 18, 10 February 2010 (2010-02-10), XP002753367,
- AN, RU et al.: "Studies on extraction and effects on hyperuricemia of quercetin from bud of sophora japonica L.", MASTER THESIS, 15 March 2013 (2013-03-15), pages 1-69, XP009515630,
- NONE: "Maikang tablet", Chinese Journal of Pharmaceuticals / Pharmaceutical industry, no. 8, 31 December 1976 (1976-12-31), pages 29-30, XP009514217, ISSN: 0255-7223

**Description**

**TECHNICAL FIELD**

The present disclosure belongs to the field of medicine, and relates to a celery seed and sophora flower bud extract which may be used for treating hyperuricemia and gout.

**BACKGROUND**

Celery seed is the seed of Apium graveolens L. which is an umbelliferous plant. Celery, also known as Petroselinum crispum or Apium graveolens, is recorded in "Lvchanyan Bencao". It is an annual or biennial umbelliferous herbaceous plant, and is cultivated throughout the country and around the world. Celery seed is often used in condiments and foods, celery seed tea has a significant effect on promoting sleep, and the extract thereof has a contractile effect on pregnant and non-pregnant uterus and may be used for treating dysmenorrhea. Traditional Chinese medicine believes that celery seed has efficacy such as dispelling Qi, diminishing swelling, inducing diuresis, eliminating obstruction, and lowering blood pressure, and it is mainly used for treating hypertension, Qi stagnation-type metritis, bronchitis, and asthma, and is applied in the clinic as an effective diuretic for treating cystitis and kidney disease. It is reported by domestic and foreign literatures that the ethanol extract of celery seed has significant pharmacological effects such as preventing and treating cardiovascular diseases, anti-cancer effect, anti-cerebral ischemia effect, reducing uric acid, and anti-inflammatory effect. Treating arthralgia with celery seed has a hundred-year history in Australia, and is considered as a traditional folk remedy. According to the description of the 1983 edition of "British Herbal Pharmacopoeia", celery seed has the effect of treating complications such as gout.

CN101007015A discloses the use of the coumarin compounds and flavonoid compounds in celery seed in the preparation of a drug for preventing and treating gout, an anti-inflammatory drug, or a health food, wherein the coumarin compounds and flavonoid compounds in celery seed are ethyl acetate extracts of celery seed. The preparation process of this invention is simple. The obtained extract has a significant anti-inflammatory effect, and is capable of reducing the amount of serum uric acid in rats. In addition, a literature (Tong Guo-hui, et al., Effect of Celery Seed Extract on Hyperuricemia in Rats, Food Science, 2008, 29(6):641) studies the effect of an ethanol extract of celery seed on the serum uric acid level in rats with hyperuricemia, and its anti-inflammatory and analgesic effects on acute gouty arthritis. The results indicate that the ethanol extract of celery seed has a certain protective effect on hyperuricemia and the impairment of renal function caused by hyperuricemia.

In a broad sense, sophora flower bud is dried flower buds and flowers of Sophora japonica L. which is a leguminous plant. It is produced in various regions of China, mainly in Loess Plateau and North China Plain. The flower buds are harvested in summer before blossom and are referred to as "sophora flower buds"; and the flowers are harvested in blossom and are referred to as "sophora flowers". After harvesting, the branches, pedicels and impurities of the inflorescences are removed, and the sophora flower buds are dried in time, used without being processed, used after frying, or used after carbonizing by stir-frying. The flower bud is oval or elliptical with a length of 2 to 6 mm and a diameter of about 2 mm; the calyx is yellow-green with several longitudinal stripes on the lower part; above the calyx are yellowish-white unbloomed petals; and the pedicel is tiny. The flower bud is lightweight, may be broken by hand twist, and is odourless and slightly bitter.

Sophora japonica L. is a deciduous tree with a height of 15 to 25 m; pinnately compound leaves alternate, the rachis is hairy, the base is swollen, the lobule is ovate-oblong and has a length of 2.5 to 7.5 cm and a width of 1.5 to 5 cm, the apex is acute, and the base is broadly cuneate, offwhite underneath, and sparsely pubescent; the panicle is terminal and campanulate with 5 denticulations, the corolla is milky white, the petal thereof is broadly heart-shaped and has short claws and purple veinlets, the edges of the wing petal and keel petal are slightly purple, 10 stamens, which are separated and unequal in length; the legume is fleshy and beadlike with a length of 2.5 to 5 cm, glabrous, and indehiscent; 1 to 6 seeds, kidney-shaped; the florescence is from July to September, and the fruit period is from September to October.

The sophora flower bud is slightly cold in nature and is bitter in taste. It has the efficacy of cooling blood and hemostasis as well as clearing liver for purging pathogenic fire, and may be used for hemafecia, hemorrhoids bleeding, bloody flux, metrorrhagia and metrostaxis, hematemesis, epistaxis, liver heat and hot eyes, headache and dizziness, and the like. The sophora flower bud belongs to a small variety, it is mainly used in pharmaceutical factories to extract "rutin", and its dosage as a traditional Chinese medicinal material is relatively small and the sales volume is not large.

As for the chemical components of the sophora flower bud, the dried bud of Sophora japonica L. contains rutoside (i.e., rutin), triterpenoid saponin (0.4%), betulin, sophoradiol, glucose, and glucuronic acid. The bud of Sophora japonica L. also contains sophorin A (14%), sophorin B (1.25%), and sophorin C (0.35%). Sophorin A is a flavonoid compound which is different from rutoside, while sophorin B and sophorin C are sterol compounds.

The pharmacological effects of the sophora flower bud are known, for example, it has the following effects:

anti-inflammatory effect: administration of rutin, which is contained in the sophora flower bud, to rat by intraperitoneal injection has a significant inhibitory effect on the inflammatory process after implanting wool balls; vitamin P-like effect: rutin has the effects of maintaining vascular resistance, lowering vascular permeability, reducing fragility, and the like, and has the effect of removing fat for a liver with fatty infiltration, and the effect of removing fat is more significant when rutin is used in combination with glutathione; antiviral effect: when the concentration of rutin is 200 $\mu$g/ml, it has the maximum inhibitory effect on vesicular stomatitis virus; inhibitory effect on aldose reductase: when the concentration of rutin is 10-5 mol/L, the inhibitory rate is 95%, and this effect is beneficial to the treatment of diabetic cataract; and effects such as eliminating phlegm and relieving a cough: quercetin contained in the sophora flower bud has a certain effect of relieving asthma; in addition, quercetin also has the effects of lowering blood pressure, enhancing capillary resistance, reducing capillary fragility, lowering blood fat, dilating coronary artery, increasing coronary blood flow, and the like.

Hyperuricemia is caused by increased production of uric acid and/or decreased secretion of renal uric acid, and is the most dangerous factor that induces gout. Reducing the blood uric acid concentration by using a drug is one of the commonly used methods for preventing gout, and such drugs include uricosuric drugs that may block the absorption of uric acid by the luminal membrane of renal tubule, and xanthine oxidase / xanthine dehydrogenase inhibitors. However, uricosuric drugs are contraindicated in patients with renal dysfunction, while use of such drugs in patients with normal renal functions may lead to the alkalization of urine. Currently, allopurinol is the only xanthine oxidase / xanthine dehydrogenase inhibitor that has been launched to the market, but it is only used in the following three kinds of patients: patients whose blood uric acid concentration cannot be reduced to below 70 mg/L by a uricosuric drug, patients who cannot tolerate a uricosuric drug, and patients who suffer from gout caused by the increased production of uric acid and suffer from renal dysfunction at the same time. In addition, allopurinol has significant adverse reactions, such as hepatitis, kidney disease, and allergic reactions.

In view of the fact that the inhibition of xanthine oxidase is the mechanism for preventing gout from occurring, researchers have fixed their eyes on the development of drugs which are safer and more effective than allopurinol. Looking back on the market of the therapeutic drugs for gout in the past two years, it is not difficult to see that a new product launched to the market in this field is rarely seen, and the market is still mainly dominated by allopurinol and benzbromarone. These two drugs are both drugs used during remission, with the former mainly inhibiting the production of uric acid, and the latter mainly promoting the excretion.

Currently, there are few varieties of gout suppressants. Colchicine, non-steroid anti-inflammatory drugs, hormones, drugs that promote the excretion of uric acid (such as probenecid, sulfinpyrazone, and benzbromarone), and drugs that inhibit the synthesis of uric acid (allopurinol) are mainly used for clinical treatment. These drugs all have defects in treatment. Poor efficacy and significant side effects have become the bottleneck of clinical applications thereof.

Clinically, a new method for treating hyperuricemia and further treating gout is still desired, and it is desired that the adverse side effects are reduced in this method under the premise of a definite therapeutic effect.

## SUMMARY

The present invention is defined by the claims. In view of the above, the technical problem to be solved by the present disclosure is to provide an extract which may be used for treating or preventing hyperuricemia and gout. The inventors of the present disclosure have unexpectedly found that an extract prepared from a combination of celery seed and sophora flower bud is not only capable of treating or preventing hyperuricemia and gout effectively, but this combination also enables the adverse side effects exhibited by the combination of the two drugs to be overcome. The present disclosure has been completed based on this finding.

The present disclosure provides a celery seed and sophora flower bud extract, wherein the celery seed and sophora flower bud extract is obtained by extracting celery seed and sophora flower bud with an alcohol solvent, wherein the ratio of the celery seed to the sophora flower bud is 2:1 to 4:1 by weight.

In a preferred embodiment of the present disclosure, provided is the celery seed and sophora flower bud extract according to the present disclosure, wherein the alcohol solvent is selected from C1 to C4 alcohol solvents, preferably methanol, ethanol, isopropanol, and n-butanol, and more preferably ethanol.

In another preferred embodiment of the present disclosure, provided is the celery seed and sophora flower bud extract according to the present disclosure, wherein the alcohol solvent is an aqueous alcohol solution, and the concentration of the aqueous alcohol solution is 50% to 80% by volume, preferably 50% to 70% by volume.

In one preferred embodiment of the present disclosure, provided is the celery seed and sophora flower bud extract according to the present disclosure, wherein the extraction method is an ultrasonic extraction method.

In another preferred embodiment of the present disclosure, provided is the celery seed and sophora flower bud extract according to the present disclosure, wherein the extraction time is 30 minutes to 60 minutes.

Another aspect of the present disclosure provides a pharmaceutical composition, comprising the celery seed and sophora flower bud extract according to the present disclosure and one or more pharmaceutically acceptable carriers.

"Pharmaceutically acceptable" described herein means that it is useful in preparation of pharmaceutical compositions that are generally safe, without biological toxicity or other undesirable toxicities, and acceptable for veterinary use and human drug use.

The "carrier" described herein refers to a diluent, an adjuvant, or an excipient which is administered together with extract. The carrier may be selected from one or more of the following: a filler, a disintegrant, a binder, and a lubricant.

The filler includes but is not limited to: starch or derivatives thereof such as corn starch, pregelatinized starch, modified starch, and the like; cellulose or derivatives thereof such as microcrystalline cellulose, ethyl cellulose, methyl cellulose, and the like; carbohydrates such as glucose, sucrose, lactose, mannitol, and sorbitol; neutral minerals such as calcium carbonate, calcium hydrogen phosphate, and the like, and a combination thereof. As used herein, the term "diluent" or "filler" is defined as an inert material used for increasing the weight and/or size of a pharmaceutical composition, and a diluent or filler may be present in the form of a substance or in the form of a mixture of compounds in the composition. Preferably, a diluent or filler is added when the amount of active ingredient(s) and other excipients is too small to obtain a tablet with a suitable size. The weight percentage of the diluent or filler necessary for the pharmaceutical composition according to the present disclosure may be determined according to conventional methods well known to those skilled in the art. Particularly, after the usage amounts of other adjuvants such as a disintegrant, a binder, a lubricant, and the like are determined, the diluent or filler is used in a proper amount according to the requirement of the size of a preparation.

The disintegrant includes but is not limited to: crosslinked polyvinyl pyrrolidone, sodium starch glycolate, croscarmellose sodium, low-substituted hydroxypropyl cellulose, and combinations thereof. Further, the pharmaceutical composition comprises 0% to 10% disintegrant, for example, 0% to 8% disintegrant, for example, 0% to 5% disintegrant, relative to the total weight of the pharmaceutical composition, and the disintegrant may also be used according to the experience of those skilled in the art. Pharmaceutical preparations in which no disintegrant or less disintegrant is used are also common. In view of the fact that the solid pharmaceutical compositions in the form of preparation in the present disclosure may not have disintegration property in certain cases, for example, when they are capsules, in these cases, the disintegrant may not be added to the solid pharmaceutical compositions of the present disclosure.

The binder includes but is not limited to: polyethylene glycol, starch, polyvinyl pyrrolidone, hydroxypropylmethyl cellulose, and combinations thereof. In view of the fact that the materials of many solid pharmaceutical preparations have a certain degree of adhesiveness by themselves and water may be used as a wetting agent to perform wet granulation, water which acts as the wetting agent may also be used as a potential binder in the present disclosure, though the water which acts as the wetting agent is removed in the final product of the solid pharmaceutical composition of the present disclosure. In addition, the materials of many solid pharmaceutical preparations have adhesiveness by themselves, thus direct tableting of the powder or direct encapsulation of the powder may be achieved. It can be seen that a binder may or may not be added to the solid pharmaceutical compositions of the present disclosure. Even if wet granulation technique is used, it is still possible that no binder is added. If added, the binder has a usage amount of 0.1% to 10%, 0.2% to 5%, or 0.5% to 2.5%, relative to the total weight of the pharmaceutical composition, and the binder may also be used according to the experience of those skilled in the art.

The function of the lubricant (including a glidant) is to enable the powder material to be formed into a preparation smoothly, for example, the powder material may be filled uniformly into a capsule shell when preparing a capsule, for example, the powder material may be filled uniformly into the mold of a tablet press when preparing a tablet, and sticking and picking are avoided. Examples of the lubricant include but are not limited to: magnesium stearate, calcium stearate, talc, starch, stearic acid, colloidal silica, and polyethylene glycol. If added, lubricant has a usage amount of 0.1% to 10%, 0.2% to 5%, or 0.2% to 2%, relative to the total weight of the pharmaceutical composition, and the lubricant may also be used according to the experience of those skilled in the art.

Another aspect of the present disclosure provides the celery seed and sophora flower bud extract according to the present disclosure or a pharmaceutical composition containing the same, for use in preventing and/or treating hyperuricemia or gout.

Although the celery seed extract and the sophora flower bud extract are known to have an effect of reducing uric acid respectively and thus are supposedly used for treating gout respectively, when the two are used in combination, the biological effect is completely unpredictable. It has been found unexpectedly that there is a risk of excessive blood pressure reduction when the two are used in combination, whereas when the two are combined in specific amounts, not only such risk of excessive blood pressure reduction may be avoided, but also a satisfactory uric acid reducing effect appears. According to the present disclosure, excessive blood pressure reduction means that the degree of blood pressure reduction after administration is greater than 15%, particularly greater than 20%, as compared with the blood pressure before administration.

The celery seed and sophora flower bud extract provided in the present disclosure may reduce uric acid and are for use in treating gout disease. Gout belongs to arthralgia syndrome in traditional Chinese medicine. With the improvement of modern living standards, the reason why people suffer from gout disease is mostly due to eating much fat and sweets and a lack of laboring. In most cases, gout belongs to beriberoid pyretic arthralgia of arthralgia syndrome in traditional Chinese medicine, which is caused by the stasis of qi and blood due to blockage of meridians and joints by wind dampness and pathogenic heat. Its symptom is the redness and swelling of joints. The disease is urgent and appears as migratory, and is accompanied by fever, dysphoria, dark urine, red tongue with yellow greasy coating, and soft and rapid pulse.

The celery seed and sophora flower bud extract provided in the present disclosure has effects of clearing heat and relieving fidgetness, inducing diuresis to alleviate edema, dispelling Qi, diminishing swelling, and eliminating obstruction. Sophora flower bud is good at clearing heat and promoting diuresis, cooling blood and hemostasis, and eliminating blood heat. When the two drugs are used in combination, wind dampness and toxic heat of meridians and joints may be eliminated so as to treat the symptoms of pain and spasm of joint-running wind.

The celery seed and sophora flower bud extract provided in the present disclosure has low toxicity and may be prepared into a variety of preparations for long-term oral maintenance dosing.

The present disclosure is described in detail below by the figures and specific Examples, but it should be understood that the figures and specific Examples are merely used to illustrate the present disclosure.

## BRIEF DESCRIPTION OF THE DRAWINGS

The figures contained in the specification and constituting a part of the specification show the exemplary Examples, features, and aspects of the present disclosure together with the specification, and are used for explaining the principles of the present disclosure.

Figure 1 is a line chart of the body weight changes of rats during a 35-day experiment.
Figure 2 is a histogram of the changes of serum uric acid level in rats during a 35-day experiment.
Figure 3 is a histogram of the changes of serum urea nitrogen level in rats during a 35-day experiment.
Figure 4 is a histogram of the changes of serum creatinine level in rats during a 35-day experiment.
Figure 5 is a histogram of the changes of serum adenosine deaminase level in rats during a 35-day experiment.
Figure 6 is a histogram of the changes of serum xanthine oxidase level in rats during a 35-day experiment.
Figure 7 is a histogram of the changes of serum guanosine deaminase level in rats during a 35-day experiment.
Figure 8 is a histogram of the 24-hour urine volumes of rats on day 35.
Figure 9 is a histogram of the urinary pH values of rats on day 35.
Figure 10 is a histogram of the concentrations of urinary uric acid of rats on day 35 of the experiment.
Figure 11 is a histogram of uric acid clearance of rats.
Figure 12 is a histogram of the concentrations of urinary creatinine of rats on day 35 of the experiment.
Figure 13 is a histogram of creatinine clearance of rats.
Figure 14 is a histogram of the concentrations of urinary urea nitrogen in of rats on day 35 of the experiment.
Figure 15 is a histogram of the right kidney weights of rats on day 35.

## DETAILED DESCRIPTION

The following Examples are for illustrative purposes only and are not intended to be and should not be construed as limiting the present disclosure in any manner. Those skilled in the art will understand that conventional variations or modifications may be made to the following Examples without departing from the scope of the present invention defined by the claims.

Celery seed and sophora flower bud were all purchased from Anguo traditional Chinese medicinal material professional market in Hebei Province.

Example 1

500 g of celery seed and 500 g of sophora flower bud were taken, 60% (V/V) ethanol which weighed 10 times the weight of the drugs was added, and ultrasonic extraction was performed three times with each time lasting 45 minutes. The extracting solution was concentrated under reduced pressure and dried under vacuum to prepare extract 1 of the present disclosure (30 g).

The extract 1 prepared above was mixed with an equal weight of dextrin uniformly and filled into capsules. The extract was contained in each capsule in such a manner that the total weight of the medicinal materials (the total weight of celery seed and sophora flower bud) was 8 g.

Example 2

1000 g of celery seed and 500 g of sophora flower bud were taken, 50% (V/V) ethanol which weighed 8 times the weight of the drugs was added, and ultrasonic extraction was performed twice with each time lasting 30 minutes. The extracting solution was concentrated under reduced pressure and dried under vacuum to prepare extract 2 of the present disclosure (26 g).

The extract 2 prepared above was mixed with an equal weight of starch uniformly and filled into capsules. The composition was contained in each capsule in such a manner that the total weight of the medicinal materials (the total weight of celery seed and sophora flower bud) was 6 g.

Example 3

2000 g of celery seed and 500 g of sophora flower bud were taken, 70% (V/V) ethanol which weighed 10 times the weight of the drugs was added, and ultrasonic extraction was performed 4 times with each time lasting 60 minutes. The extracting solution was concentrated under reduced pressure and dried under vacuum to prepare extract 3 of the present disclosure (44 g).

The extract 3 prepared above was mixed with an equal weight of dextrin uniformly and filled into capsules. The composition was contained in each capsule in such a manner that the total weight of the medicinal materials (the total weight of celery seed and sophora flower bud) was 5 g.

Example 4 Preparation of tablets

Using the extract 1 prepared in Example 1, tablets were prepared according to the following formulation:

| | |
|---|---|
| extract 1 | 100 mg |
| microcrystalline cellulose (diluent) | 150 mg |
| HPMC (binder) | 5 mg |
| crospovidone (disintegrant) | 20 mg |
| aerosil (lubricant) | 5 mg |

Extract 1 and microcrystalline cellulose were mixed, an HPMC solution (5% aqueous solution) was added, and the mixture was granulated and dried. Crospovidone and aerosol were added to the dry granules, and the mixture was mixed uniformly and tableted to obtain the tablets.

Example 5 Preparation of pellets

Using the extract 2 prepared in Example 2, pellets (capsulated) were prepared according to the following formulation:

| | |
|---|---|
| extract 2 | 100 mg |
| microcrystalline cellulose (diluent) | 150 mg |
| HPMC (binder) | 5 mg |
| crospovidone (disintegrant) | 20 mg |
| aerosil (lubricant) | 5 mg |

Preparation was conducted according to the following method:

(1) a prescribed amount of extract 2 was weighed, microcrystalline cellulose and crospovidone were added, and the mixture was smashed and mixed uniformly;
(2) an HPMC solution (5% aqueous solution) was added;
(3) pellets were prepared by using an extrusion-spheronization method: the mixed materials were extruded into bars at an extrusion speed of 10-40 r/min, the extrudate was transferred into a spheronization apparatus, and the extrudate was first cut at a rotation speed of 40 Hz to 70 Hz and then spheronized for 10 to 20 minutes at a rotation speed of 30 Hz to 50 Hz to prepare the pellets; and
(4) the prepared pellets were transferred to a drying room and subjected to drying. The drying condition was 30 ± 2 °C and the drying time was 12 hours. The pellets were encapsulated after drying.

Experimental example 1: Hypotensive effect of the extracts of the present disclosure

Experimental animals

SD rats, male, purchased from Beijing SPF Animal Experimental Center, laboratory animal quality certificate number: 11401500006019, animal house license number: SCXK (Beijing) 2011-0004.

Experimental method

SD rats were randomly divided into groups with at least 10 animals in each group. 5 days prior to administration, the blood pressure of each animal was measured (RBP-1 type tail arterial blood pressure meter for rat, China-Japan Friendship Institute of Clinical Medical Science) at 10:00 am every day. The mean value of the blood pressures of all animals in each group in 5 days was taken as the blood pressure prior to administration (B0). The administration groups of the extracts of the present disclosure were administered once at 10:00 am every day (the blood pressure was measured before administration) at a dose of 5 g/kg/day in terms of the total weight of the medicinal materials (the total weight of celery seed and sophora flower bud). After 10 days of continuous administration, the mean value of the last measured blood pressures (B1) was calculated. The mean degree of blood pressure reduction in rats was calculated according to the following formula:

$$\text{Mean degree of blood pressure reduction (\%)} = [(B0 - B1) \div B0] \times 100\%.$$

Results

The mean degree of blood pressure reduction (%) of extract 1 was 8%; the mean degree of blood pressure reduction (%) of extract 2 was 12%; and the mean degree of blood pressure reduction (%) of extract 3 was 9%.

Experimental example 2: Uric acid reducing effect of the extracts of the present disclosure

1) Experimental animals

SD rats, male, weighing $200 \pm 20$ g, 84 rats in total; purchased from Beijing SPF Animal Experimental Center, laboratory animal quality certificate number: 11401500006019, animal house license number: SCXK (Beijing) 2011-0004.

2) Test reagents

Positive control drug: benzbromarone, produced by Heumann Pharma, specification: 50 mg/tablet; batch number: 150201.
Assay kits: Uric Acid (UA) Assay Kit: from BioSino Bio-Technology & Science Inc.; Creatinine (CR) Assay Kit: from Nanjing Jiancheng Bioengineering Institute; Xanthine Oxidase (XOD) Assay Kit: from Nanjing Jiancheng Bioengineering Institute; Adenosine Deaminase (ADA) Assay Kit: from Nanjing Jiancheng Bioengineering Institute; Urea Nitrogen (BUN) Assay Kit: from Nanjing Jiancheng Bioengineering Institute; Guanine Deaminase (GuDa) Assay Kit: from Nanjing Jiancheng Bioengineering Institute.
Modeling agents: D-fructose, AMRESCO Inc., the USA, batch number: 2563C076; hypoxanthine, purchased from Sigma Inc., batch number: 060M1199V; nicotinic acid, purchased from Beijing BioDee BioTech Corporation, Ltd., batch number: N4126.

3) Experimental instruments

LJX-II centrifuge (Shanghai Medical Analytical Instrument Factory), Low-temperature and high-speed centrifuge (Sigma Inc.), SHZ88-1 type benchtop water-bathing thermostatic shaker water bath (Taicang Guangming Experimental Analytical Instrument Factory), SHIMADZUBI-220H electronic balance, Sunrise microplate reader (Tecan Group, Tecan Austria GmbH).

4) Modeling of animals

For the modeling method of the experiment, the following were referred to: the published research paper "Effect of high fructose drinking water on uric acid level in rats and the underlying pathological mechanism" (Li Li-yu, Lin Zhi-jian, Zhang Bing, et al, Effect of high fructose drinking water on uric acid level in rats and the underlying pathological mechanism, Chinese Journal of Clinical Nutrition, 2014, 22(6):368-374), and the literatures "Preliminary study on hyperuricemia model in mice" (Jin Shen-rui, Zheng Jun, Liu Shao-tang, Preliminary study on hyperuricemia model in mice, Journal of Chengdu University of Traditional Chinese Medicine, 1999, 22(01):50-51+65) and "Eeffcts of Rebixiao granules on blood uric acid in patients with repeatedly attacking acute gouty arthritis" (Ji Wei, Zhu Xuan-xuan, Tan Wen-feng, Lu Yan. Effects of Rebixiao granules on blood uric acid in patients with repeatedly attacking acute gouty arthritis. Chinese Journal of Integrative Medicine,2005, 11(1):15-21.)

5) Grouping and administration

SD rats were stratified by weight using random number table method, and each layer was divided into a normal group (10 rats), a fructose modeling group (50 rats) and a purine modeling group (24 rats) using random number table method. Tap water was administered to the normal group; a 10% (w/v) fructose solution was administered to the fructose modeling group for modeling; and for the purine modeling group, tap water was administered for drinking, 500 mg/kg of hypoxanthine + 100 mg/kg of nicotinic acid was administered by gastric perfusion according to body weight once a day, and the volume of perfusion was 10 ml/kg. The food and water consumption of rats were recorded daily, the body weights were weighed every 7 days, and blood was taken by shearing the tail tip on the next day of weighing.

When the blood uric acid level of the fructose modeling group was significantly higher than that of the normal group (P < 0.01), the fructose modeling group was divided into a model group, a benzbromarone control group, and large-, medium- and small-dose groups of the extract of the present disclosure (10 rats in each group) using random number table method, and the baselines of the body weight and blood uric acid level of rats in each group were kept consistent after grouping. Among them, 0.5% CMC-Na was administered to the normal group and the model group by gastric perfusion, and respective drugs dissolved in 0.5% CMC-Na were administered to the other administration groups by gastric perfusion. The perfusion was performed once every morning. The dosages of administration were as follows: the dosage of benzbromarone was 20 mg/kg, the large-, medium- and small-dosages of the extract of the present disclosure were 1.9 g/kg, 0.95 g/kg, and 0.475 g/kg, respectively, the volume of administration was 15 ml/kg, and the experiment lasted for 35 days in total. The dosages of administration of rats in each group were as shown in Table 1 below. During the experiment, since there was no significant difference in blood uric acid level between the purine modeling group and the normal group from beginning to end, the modeling of the hyperuricemia model in rats was not successful, therefore, the rats were not further grouped.

Table 1 Gastric perfusion dosages of rats in each group

| Group | Number of animals | Drug | Solvent | Dosage of administration | Volume of gastric perfusion |
|---|---|---|---|---|---|
| Normal group | 10 | - | 0.5% CMC-Na | - | 1.0 ml/100 g |
| Model group | 10 | - | 0.5% CMC-Na | - | 1.0 ml/100 g |
| Benzbromarone control group | 10 | Benzbromarone | 0.5% CMC-Na | 20 mg/kg | 1.0 ml/100 g |
| Large-dose group | 10 | Extract 1 | 0.5% CMC-Na | 1.9 g/kg | 1.0 ml/100 g |
| Medium-dose group | 10 | Extract 1 | 0.5% CMC-Na | 0.95 g/kg | 1.0 ml/100 g |
| Small-dose group | 10 | Extract 1 | 0.5% CMC-Na | 0.475 g/kg | 1.0 ml/100 g |

6) Detection indices and determination methods

Every 7 days during the experiment, the body weights of the animals were weighed and recorded, blood was taken from the tail vein, and serum was separated by centrifugation. The uric acid (UA), urea nitrogen (BUN), creatinine (CRE), xanthine oxidase (XOD), adenosine deaminase (ADA) and guanine deaminase (GuDa) in serum were detected. On day 35 of the experiment, the 24-hour urine of the rats was collected, the urine volume was measured, the pH value was determined, and the urinary uric acid level, urinary creatinine level, and urinary urea nitrogen level were detected. After the experiment was terminated, the rats were sacrificed, and the right kidneys were taken and weighed.

The specific determination method of each index was as follows.

① Detecting serum uric acid and urinary uric acid by uricase colorimetric method: uric acid was catalyzed by uricase to produce allantoin and hydrogen peroxide ($H_2O_2$); $H_2O_2$, 4-aminoantipyrine (4-AAP) and N-ethyl-N-(2-hydroxy-3-sulfopropyl)-3-methylaniline (TOOS) were catalyzed by peroxidase (POD) to produce a colored quinone imine compound, and the produced amount thereof was directly proportional to the concentration of uric acid. A serum or urine sample was taken. According to the operating instruction of Uric Acid Assay Kit, under the conditions of a cuvette light path of 1 cm and a wavelength of 540 nm to 560 nm, a blank tube was used for zero calibration; the absorbance was determined by a UV spectrophotometer, and the uric acid value was obtained.

② Detecting serum creatinine and urinary creatinine by picric acid colorimetric method: Jaffe reaction occurred between the creatinine in the supernatant of serum from which protein was removed or the creatinine in clear urine and picric acid to produce an orange adduct. A serum or urine sample was taken. According to the operating instruction of Creatinine Assay Kit, under the conditions of a cuvette light path of 1 cm and a wavelength of 510 nm, double distilled water was used for zero calibration; the absorbance was determined by a UV spectrophotometer, and the creatinine value was obtained.

③ Detecting serum urea nitrogen and urinary urea nitrogen by urease method: urea was hydrolyzed under the effect of urease to produce ammonia ions and carbon dioxide, the ammonia ions formed a blue substance with a phenol developer in an alkaline medium, and the amount of the substance produced was directly proportional to the content of urea. A serum or urine sample was taken. According to the operating instruction of Urea Nitrogen Assay Kit, under the conditions of a cuvette light path of 1 cm and a wavelength of 640 nm, double distilled water was used for zero calibration; the absorbance was determined by a UV spectrophotometer, and the urea nitrogen value was obtained.

④ Detecting xanthine oxidase (XOD) by enzymatic colorimetric method: hypoxanthine could be catalyzed by xanthine oxidase to produce xanthine, and superoxide anion radicals were produced at the same time. In the presence of an electron acceptor and a developer, a purple red conjugate was produced, and the activity of xanthine oxidase could be calculated according to the amount of the latter produced. A serum sample was taken. According to the operating instruction of Xanthine Oxidase Assay Kit, under the conditions of a cuvette the light path of 1 cm and a wavelength of 530 nm, double distilled water was used for zero calibration; the absorbance was determined by a UV spectrophotometer, and the activity of xanthine oxidase was obtained.

⑤ Detecting adenosine deaminase (ADA) by enzymatic colorimetric method: adenine riboside was catalyzed by adenosine deaminase to hydrolyze and produce hypoxanthine riboside and ammonia, and the produced ammonia was developed. Therefore, the activity of serum adenosine deaminase could be calculated. A serum sample was taken. According to the operating instruction of Adenosine Deaminase Assay Kit, under the conditions of a cuvette light path of 1 cm and a wavelength of 640 nm, double distilled water was used for zero calibration; the absorbance was determined by a UV spectrophotometer, and the activity of adenosine deaminase was obtained.

⑥ Detecting guanine deaminase (GuDa) by single enzyme reagent method: guanine was deaminized to form xanthine under the effect of guanine deaminase, then xanthine was catalyzed by xanthine oxidase to release superoxide anions, so that nitrotetrazolium blue chloride (NBT) was reduced to purple red. A serum sample was taken. According to the operating instruction of Guanine Deaminase Assay Kit, under the conditions of a cuvette light path of 0.5 cm and a wavelength of 540 nm, double distilled water was used for zero calibration; the absorbance was determined by a UV spectrophotometer, and the activity of guanine deaminase was obtained.

7) Statistics and analysis of the results

The results were represented as mean $\pm$ standard deviation ($\bar{x} \pm s$), the body weights and the serum biochemical indices were respectively calculated by SPSS 17.0 statistical software to obtain the mean and the standard deviation of each group, and ANOVA analysis was performed.

8) Results

8.1) Body weight changes

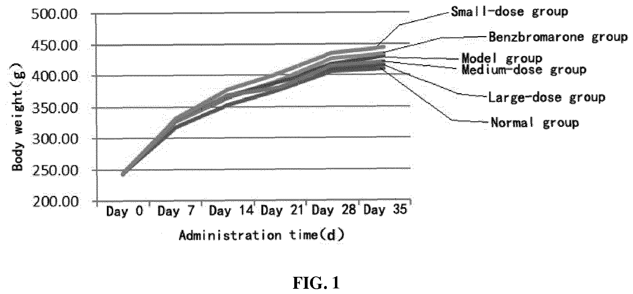

As compared with the normal group, on day 21 of the experiment, the body weights of the rats in the model group increased significantly (P < 0.05). As compared with the model group, during the experiment, no significant difference was observed in body weight between rats in each administration group. See Table 2 and Figure 1.

Table 2 Table of the body weight changes of rats during a 35-day experiment (n=10, ($\bar{x} \pm$ s), unit: g)

| Group | Modeling period | | | Administration period | | |
|---|---|---|---|---|---|---|
| | Day 0 | Day 7 | Day 14 | Day 21 | Day 28 | Day 35 |
| Normal group | 243.61±8.88 | 316.83±11.40 | 353.22±15.68 | 376.73±15.51 | 405.57±16.50 | 409.62±20.52 |
| Model group | 243.25±9.45 | 326.18±14.57 | 364.32±12.45 | 390.99±16.06* | 417.42±29.82 | 429.81±31.19 |
| Benzbromarone group | 243.40±7.63 | 325.46±15.10 | 365.30±20.19 | 391.83±19.39 | 425.86±22.33 | 433.88±27.07 |
| Large-dose group | 243.13±10.65 | 330.13±14.15 | 366.98±18.67 | 389.00±25.91 | 409.11±22.76 | 415.53±21.42 |
| Medium-dose group | 243.62±9.27 | 326.98±16.24 | 367.38±19.63 | 381.15±18.23 | 415.22±24.52 | 420.78±25.99 |
| Small-dose group | 243.20±11.44 | 330.60±16.24 | 376.54±19.93 | 403.80±18.23 | 434.62±24.52 | 443.78±25.99 |

8.2) Change of serum uric acid (UA) level

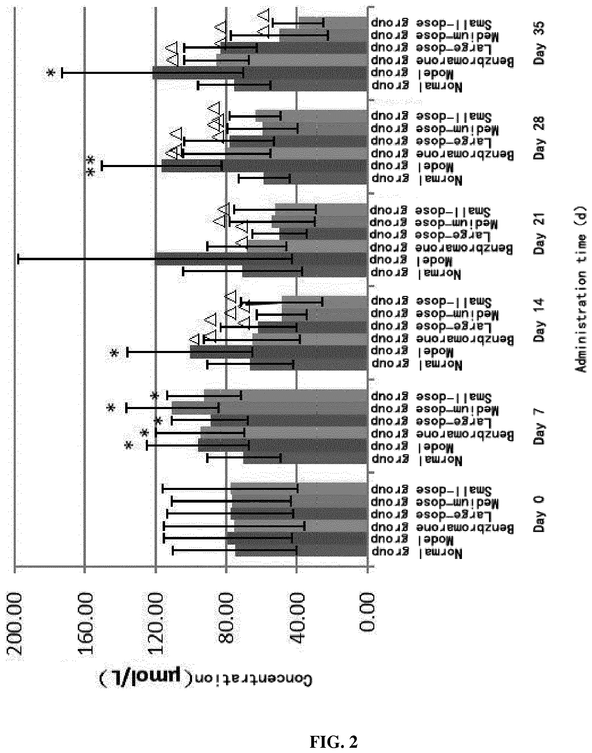

As compared with the normal group, on day 7, day 14, day 28, and day 35 of the experiment, the serum UA level of the model group showed a significant increase ($P < 0.05$ or $P < 0.01$), indicating that the hyperuricemia model was established successfully. As compared with the model group, on day 14, day 28, day 35 of the experiment, the serum UA level of the benzbromarone group showed a significant decrease ($P < 0.05$); and on day 14, day 21, day 28, and day 35 of the experiment, the serum UAlevels in the large-, medium- and small-dose groups all showed a significant decrease ($P < 0.05$ or $P < 0.01$). See Table 3 and Figure 2.

Table 3 Table of the changes of serum uric acid level in rats during a 35-day experiment (n=10, ($\bar{x} \pm s$), unit: $\mu$mol/L)

| Group | Modeling period | | | Administration period | | |
|---|---|---|---|---|---|---|
| | Day 0 | Day 7 | Day 14 | Day 21 | Day 28 | Day 35 |
| formal group | 75.18±35.18 | 70.15±20.62 | 66.68±24.43 | 70.79±33.60 | 58.79±14.41 | 75.51±20.76 |
| Model group | 79.09±36.13 | 95.99±28.69* | 100.50±35.51* | 120.57±77.60 | 116.44±33.96** | 121.79±51.31* |
| Benzbromarone group | 75.83±38.77 | 94.75±25.26* | 65.57±27.48$^\Delta$ | 68.45±22.20 | 79.65±24.68$^\Delta$ | 85.66±18.37$^\Delta$ |
| Large-dose group | 77.79±35.87 | 89.22±21.48* | 61.81±21.72$^{\Delta\Delta}$ | 49.87±15.60$^{\Delta\Delta}$ | 78.28±25.43$^{\Delta\Delta}$ | 83.26±20.39$^\Delta$ |
| Medium-dose group | 77.14±33.96 | 110.57±26.17** | 48.67±14.22$^{\Delta\Delta}$ | 54.17±23.95$^\Delta$ | 59.50±19.77$^{\Delta\Delta}$ | 49.88±27.56$^{\Delta\Delta}$ |
| Small-dose group | 77.79±38.41 | 92.79±20.77* | 48.67±23.09$^{\Delta\Delta}$ | 52.40±23.18$^\Delta$ | 63.61±14.57$^{\Delta\Delta}$ | 39.20±14.49$^{\Delta\Delta}$ |

Note: P*<0.05, P**<0.01, as compared with the normal group; and P$^\Delta$<0.05, P$^{\Delta\Delta}$<0.01, as compared with the model group.

8.3) Changes of serum creatinine (CRE) level and urea nitrogen (BUN) level

As compared with the normal group, during the experiment, the serum BUN level of the model group showed no significant difference (P > 0.05). As compared with the model group, on day 21 and day 35 of the experiment, the serum BUN level of the large-dose group showed a significant decrease (P < 0.05 or P < 0.01), on day 35 of the experiment, the serum BUN levels in the medium- and small-dose groups showed a significant decrease (P < 0.05).

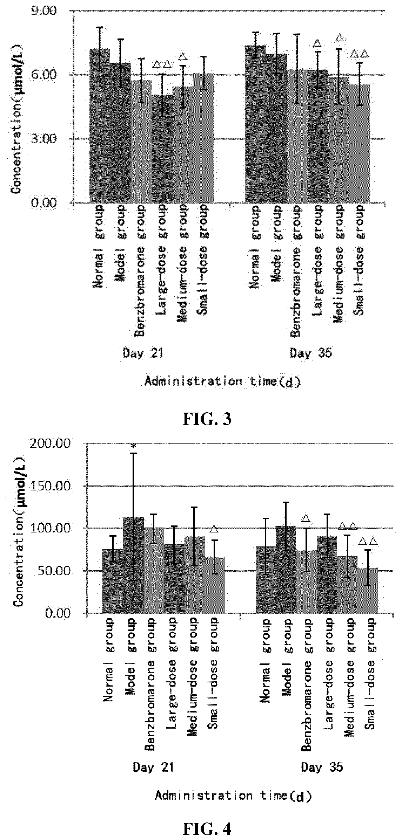

As compared with the normal group, on day 21 of the experiment, the serum CRE level of the model group showed a significant increase (P < 0.05). As compared with the model group, on day 35 of the experiment, the serum CRE level of the benzbromarone group showed a significant decrease (P < 0.05); on day 21 of the experiment, the serum CRE level of the small-dose group showed a significant decrease (P < 0.05); and on day 35 of the experiment, the serum CRE levels in the medium- and small-dose groups showed an extremely significant decrease (P < 0.01). See Table 4 and Figures 3 and 4.

Table 4 Table of the changes of serum creatinine level and serum urea nitrogen level in rats during a 35-day experiment (n=10, ( ), unit: $\mu$mol/L

| Group Time | Serum urea nitrogen | | Serum creatinine | |
|---|---|---|---|---|
| | Day 21 | Day 35 | Day 21 | Day 35 |
| Normal group | 7.21$\pm$1.01 | 7.38$\pm$0.61 | 75.73$\pm$15.16 | 78.79$\pm$32.91 |
| Model group | 6.54$\pm$1.13 | 6.99$\pm$0.94 | 113.49$\pm$75.34* | 102.37$\pm$28.74 |
| Benzbromarone group | 5.73$\pm$1.02 | 6.27$\pm$1.62 | 99.21$\pm$17.50 | 74.54$\pm$25.76$^\Delta$ |
| Large-dose group | 5.04$\pm$1.00$^{\Delta\Delta}$ | 6.22$\pm$0.84$^\Delta$ | 81.05$\pm$22.08 | 90.89$\pm$25.48 |
| Medium-dose group | 5.44$\pm$0.98$^\Delta$ | 5.92$\pm$1.30$^\Delta$ | 91.05$\pm$34.18 | 67.32$\pm$24.52$^{\Delta\Delta}$ |
| Small-dose group | 6.08$\pm$0.77 | 5.56$\pm$1.01$^{\Delta\Delta}$ | 66.67$\pm$19.95$^\Delta$ | 53.60$\pm$20.88$^{\Delta\Delta}$ |

Note: P* < 0.05, P** < 0.01, as compared with the normal group; and P$^\Delta$ < 0.05, P$^{\Delta\Delta}$ < 0.01, as compared with the model group.

8.4) Changes of the activity levels of serum adenosine deaminase (ADA), serum xanthine oxidase (XOD), and serum guanosine deaminase (GuDa)

As compared with the normal group, during the experiment, the serum ADA level of the model group showed no significant difference (P > 0.05). As compared with the model group, on day 21 of the experiment, the serum ADA level of the large-dose group showed a significant decrease (P < 0.05), and on day 35 of the experiment, the serum ADA levels in the medium- and small-dose groups showed a significant decrease (P < 0.05 or P < 0.01).

As compared with the normal group, on day 21 of the experiment, the serum XOD level of the model group showed a significant increase (P < 0.05). As compared with the model group, on day 35 of the experiment, the serum XOD level of the small-dose group showed a significant decrease (P < 0.01). During the experiment, the serum XOD levels in the large- and medium-dose groups showed no significant difference (P > 0.05).

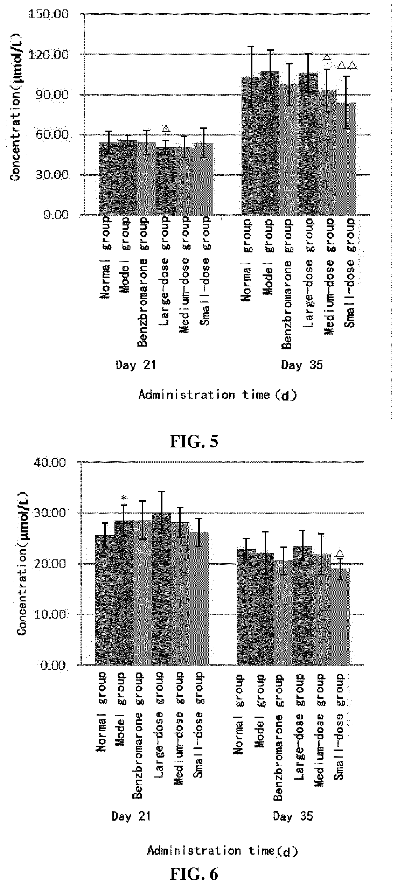

As compared with the normal group, during the experiment, the serum GuDa level of the model group showed no significant difference (P > 0.05). As compared with the model group, on day 21 of the experiment, the serum GuDa level of the benzbromarone group showed a significant decrease (P < 0.05), on day 35 of the experiment, the serum GuDa levels in the medium- and small-dose groups showed a significant decrease (P < 0.05 or P < 0.01), and during the experiment, the serum GuDa level of the large-dose group showed no significant difference (P > 0.05). See Tables 5, 6 and 7 and Figures 5, 6 and 7.

Table 5 Table of the changes of serum adenosine deaminase level in rats during a 35-day experiment (n=10, $\bar{x} \pm s$, unit: U/ml)

| Group | Day 21 | Day 35 |
|---|---|---|
| Normal group | 54.22 $\pm$ 8.18 | 103.27 $\pm$ 22.65 |
| Model group | 55.66 $\pm$ 3.89 | 107.05 $\pm$ 16.23 |
| Benzbromarone group | 54.22 $\pm$ 8.72 | 97.69 $\pm$ 15.32 |
| Large-dose group | 50.47 $\pm$ 5.48$^\Delta$ | 106.20 $\pm$ 14.45 |
| Medium-dose group | 51.02 $\pm$ 7.94 | 93.16 $\pm$ 15.60$^\Delta$ |

(continued)

| Group | Day 21 | Day 35 |
|---|---|---|
| Small-dose group | 53.75 ± 11.03 | 84.04 ± 19.51$^{\Delta\Delta}$ |

Note: P* < 0.05, P** < 0.01, as compared with the normal group; and P$^{\Delta}$ < 0.05, P$^{\Delta\Delta}$ < 0.01, as compared with the model group.

Table 6 Table of the changes of serum xanthine oxidase level in rats during a 35-day experiment (n=10, $\overline{x} \pm s$, unit: U/L

| Group | Day 21 | Day 35 |
|---|---|---|
| Normal group | 25.67 ± 2.36 | 22.95 ± 2.11 |
| Model group | 28.63 ± 3.03* | 22.14 ± 4.15 |
| Benzbromarone group | 28.66 ± 3.75 | 20.61 ± 2.67 |
| Large-dose group | 30.14 ± 4.06 | 23.60 ± 2.95 |
| Medium-dose group | 28.18 ± 2.84 | 21.88 ± 4.00 |
| Small-dose group | 26.24 ± 2.79 | 19.04 ± 2.05$^{\Delta}$ |

Note: P* < 0.05, P** < 0.01, as compared with the normal group; and P$^{\Delta}$ < 0.05, P$^{\Delta\Delta}$ < 0.01, as compared with the model group.

Table 7 Table of the changes of serum guanosine deaminase level in rats during a 35-day experiment (n=10, $\overline{x} \pm s$, unit: U/L)

| Group | Day 21 | Day 35 |
|---|---|---|
| Normal group | 22.81 ± 4.44 | 12.17 ± 4.45 |
| Model group | 24.90 ± 6.51 | 17.97 ± 12.08 |
| Benzbromarone group | 29.46 ± 3.57$^{\Delta}$ | 11.46 ± 4.83 |
| Large-dose group | 24.71 ± 4.33 | 11.18 ± 6.03 |
| Medium-dose group | 27.01 ± 5.35 | 10.00 ± 3.74$^{\Delta}$ |
| Small-dose group | 26.78 ± 5.49 | 7.91 ± 3.96$^{\Delta}$ |

Note: P* < 0.05, P** < 0.01, as compared with the normal group; and P$^{\Delta}$<0.05, P$^{\Delta\Delta}$<0.01, as compared with the model group.

8.5) 24-hour urine volumes and urinary pH values of rats

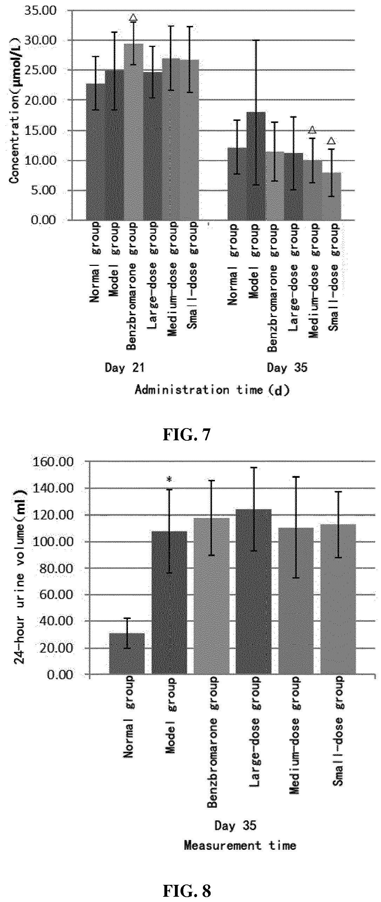

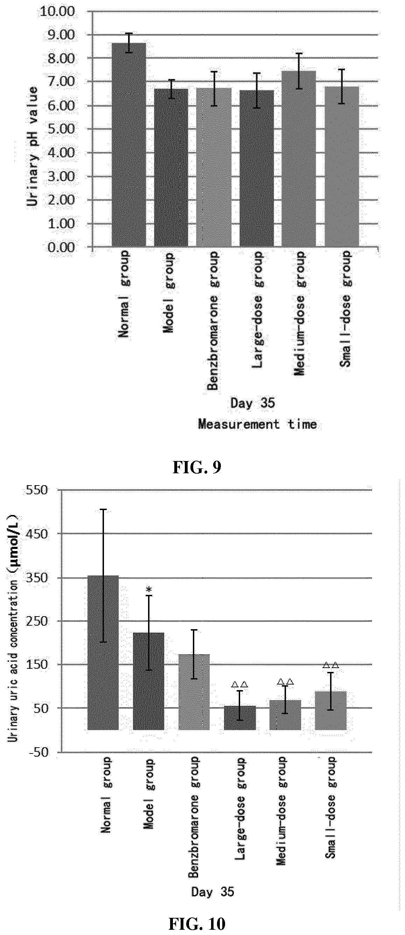

On day 35 of the experiment, as compared with the normal group, the 24-hour urine volume in the model group showed an extremely significant increase (P < 0.01); and as compared with the model group, there was no significant difference between each administration group (P > 0.05). As compared with the normal group, the urinary pH value in the model group showed an extremely significant decrease (P < 0.01); and as compared with the model group, the urinary pH value in the medium-dose group showed a significant increase (P < 0.05). See Table 8, Table 9 和 Figure 8, Figure 9.

Table 8 Table of the 24-hour urine volumes of rats on Day 35 (n=10, $\overline{x} \pm s$, unit: ml)

| Group | Urine volume |
|---|---|
| Normal group | 31.10 ± 11.08 |
| Model group | 107.88 ± 31.27** |
| Benzbromarone group | 117.80 ± 31.17 |
| Large-dose group | 124.10 ± 31.17 |
| Medium-dose group | 110.67 ± 38.04 |

(continued)

| Group | Urine volume |
|---|---|
| Small-dose group | 112.89 ± 24.73 |

Note: P* < 0.05, P** < 0.01, as compared with the normal group; and P$^\Delta$ < 0.05, P$^{\Delta\Delta}$ < 0.01, as compared with the model group.

Table 9 Table of the urinary pH values of rats on Day 35 (n=10, $\overline{x} \pm s$)

| Group | pH |
|---|---|
| Normal group | 8.64 ± 0.42 |
| Model group | 6.69 ± 0.37** |
| Benzbromarone group | 6.70 ± 0.71 |
| Large-dose group | 6.62 ± 0.72 |
| Medium-dose group | 7.45 ± 0.76$^{\Delta\Delta}$ |
| Small-dose group | 6.79 ± 0.71 |

Note: P* < 0.05, P** < 0.01, as compared with the normal group; and P$^\Delta$ < 0.05, P$^{\Delta\Delta}$ < 0.01, as compared with the model group.

8.6) Changes of urinary uric acid (UUA) level, uric acid clearance level, urinary creatinine (UCRE) level, creatinine clearance level, and urinary urea nitrogen (UBUN) level

On day 35 of the experiment, as compared with the normal group, the urinary UA level of the model group decreased significantly (P < 0.05); and as compared with the model group, the urinary UA levels in the large-, medium- and small-dose groups showed significant decrease (P < 0.01). As compared with the normal group, the urinary CRE level of the model group decreased significantly (P < 0.01); and as compared with the model group, the urinary CRE level of the medium-dose group decreased significantly (P < 0.05). As compared with the normal group, the urinary BUN level of the model group decreased significantly (P < 0.01); and as compared with the model group, there was no significant difference between each administration group (P > 0.05).

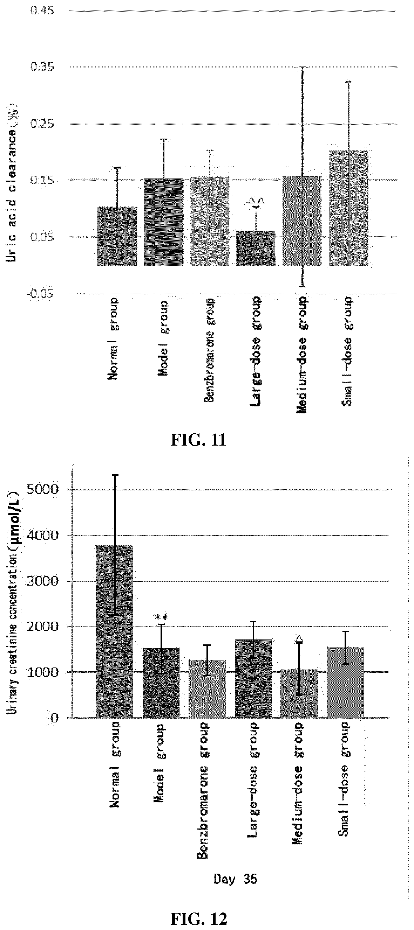

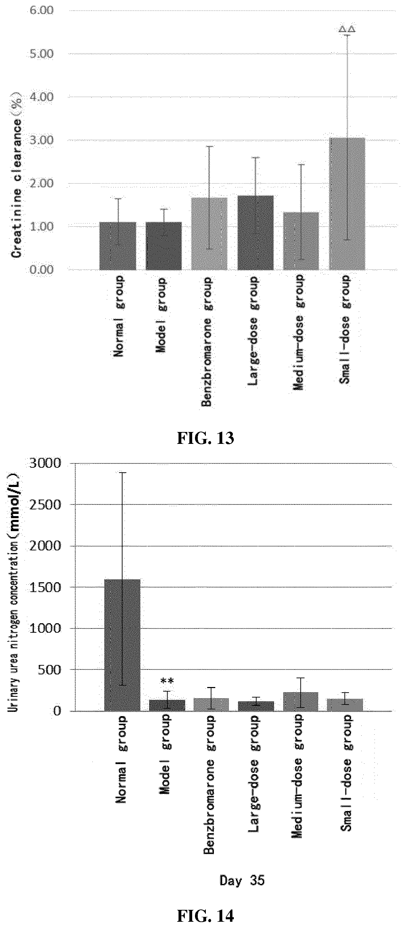

The uric acid clearance and the creatinine clearance of rats were calculated. As compared with the normal group, the uric acid clearance in the model group showed no significant change (P > 0.05); and as compared with the model group, the uric acid clearance in the large-dose group showed a significant decrease (P < 0.01). As compared with the normal group, the creatinine clearance in the model group showed no significant change (P > 0.05); and as compared with the model group, the creatinine clearance in the small-dose group showed a significant increase (P < 0.01). See Table 10, Table 11, Table 12, Table 13, Table 14, and Figure 10, Figure 11, Figure 12, Figure 13, and Figure 14.

Table 10 Table of the concentrations of urinary uric acid of rats on day 35 of the experiment (n=10, $\overline{x} \pm s$, unit: μmol/L)

| Group | UUA |
|---|---|
| Normal group | 353.69 ± 153.18 |
| Model group | 223.03 ± 85.30* |
| Benzbromarone group | 173.57 ± 56.65 |
| Large-dose group | 56.15 ± 33.91$^{\Delta\Delta}$ |
| Medium-dose group | 69.26 ± 31.31$^{\Delta\Delta}$ |
| Small-dose group | 88.64 ± 42.81$^{\Delta\Delta}$ |

Note: P* < 0.05, P** < 0.01, as compared with the normal group; and P$^\Delta$ < 0.05, P$^{\Delta\Delta}$ < 0.01, as compared with the model group.

Table 11 Table of uric acid clearance of rats

| Group | Uric acid clearance |
|---|---|
| Normal group | 0.104 ± 0.067 |

(continued)

| Group | Uric acid clearance |
|---|---|
| Model group | 0.153 ± 0.070 |
| Benzbromarone group | 0.155 ± 0.047 |
| Large-dose group | 0.062 ± 0.042$^{\Delta\Delta}$ |
| Medium-dose group | 0.157 ± 0.194 |
| Small-dose group | 0.202 ± 0.122 |

Note: P* < 0.05, P** < 0.01, as compared with the normal group; and P$^{\Delta}$ < 0.05, P$^{\Delta\Delta}$ < 0.01, as compared with the model group.

Table 12 Table of the concentrations of urinary creatinine of rats on day 35 of the experiment (n=10, $\bar{x} \pm s$, unit: μmol/L)

| Group | UCR |
|---|---|
| Normal group | 3787.71 ± 1529.84 |
| Model group | 1511.71 ± 531.31** |
| Benzbromarone group | 1256.25 ± 332.40 |
| Large-dose group | 1706.60 ± 401.01 |
| Medium-dose group | 1061.89 ± 567.20$^{\Delta}$ |
| Small-dose group | 1531.20 ± 354.08 |

Note: P* < 0.05, P** < 0.01, as compared with the normal group; and P$^{\Delta}$ < 0.05, P$^{\Delta\Delta}$ < 0.01, as compared with the model group.

Table 13 Table of creatinine clearance of rats

| Group | Creatinine clearance |
|---|---|
| Normal group | 1.11 ± 0.53 |
| Model group | 1.10 ± 0.31 |
| Benzbromarone group | 1.67 ± 1.18 |
| Large-dose group | 1.72 ± 0.88 |
| Medium-dose group | 1.34 ± 1.10 |
| Small-dose group | 3.06 ± 2.36$^{\Delta\Delta}$ |

Note: P* < 0.05, P** < 0.01, as compared with the normal group; and P$^{\Delta}$ < 0.05, P$^{\Delta\Delta}$ < 0.01, as compared with the model group.

Table 14 Table of the concentrations of urinary urea nitrogen of rats on day 35 of the experiment (n=10, $\bar{x} \pm s$, unit: mmol/L)

| Group | UBUN |
|---|---|
| Normal group | 1600.05 ± 1289.34 |
| Model group | 139.49 ± 101.67** |
| Benzbromarone group | 156.36 ± 133.40 |
| Large-dose group | 119.93 ± 48.73 |
| Medium-dose group | 224.18 ± 179.18 |
| Small-dose group | 147.96 ± 71.15 |

Note: P* < 0.05, P** < 0.01, as compared with the normal group; and P$^{\Delta}$ < 0.05, P$^{\Delta\Delta}$ < 0.01, as compared with the model group.

8.7) Right kidney weights of rats

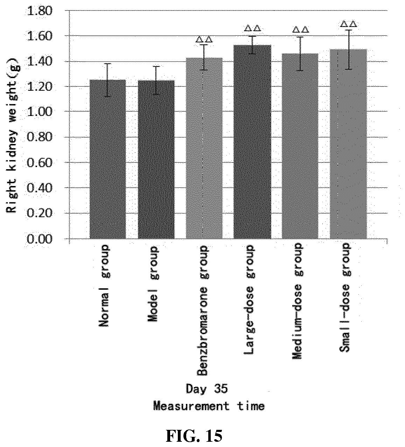

On day 35 of the experiment, as compared with the normal group, the kidney weight of the model group showed no difference (P > 0.05); and as compared with the model group, the kidney weight of each administration group increased significantly (P < 0.01). See Table 15 and Figure 15.

Table 15 Table of the right kidney weights of rats on Day 35 (n=10, $\bar{x} \pm s$, unit: g)

| Group | Weight |
|---|---|
| Normal group | $1.25 \pm 0.13$ |
| Model group | $1.25 \pm 0.11$ |
| Benzbromarone group | $1.43 \pm 0.10^{\Delta\Delta}$ |
| Large-dose group | $1.53 \pm 0.07^{\Delta\Delta}$ |
| Medium-dose group | $1.46 \pm 0.13^{\Delta\Delta}$ |
| Small-dose group | $1.49 \pm 0.15^{\Delta\Delta}$ |

Note: $P^* < 0.05$, $P^{**} < 0.01$, as compared with the normal group; and $P^{\Delta} < 0.05$, $P^{\Delta\Delta} < 0.01$, as compared with the model group.

9) Conclusion

A stable hyperuricemia model was established successfully in the present disclosure by high-fructose water intake for rats. During the experiment, biochemical indices such as *in vivo* serum uric acid, serum creatinine and serum urea nitrogen of rats were observed dynamically, the uric acid reducing effects of different doses of the extract of the present disclosure were observed, and the mechanism of action was investigated from two pathways: uric acid production and uric acid excretion.

The results of the experiment indicated that the extracts of the present disclosure were capable of reducing uric acid production and reducing the blood uric acid level in rats by reducing the activities of the enzymes (XOD, ADA, and GuDa) which were associated with uric acid production; might improve the renal excretion function of rats; and at the same time were capable of promoting renal uric acid excretion to a certain extent. Among the three dosages used in this experiment, the extracts of the present disclosure in the small-dose extract (0.475g/kg) group had the best effect of reducing blood uric acid level in rats, and might be used as a reference for clinical administration dosage.

Experimental example 3 Effects of extracts with different ratios of medicinal materials Different extracts were prepared in accordance with the ratios of the medicinal materials in Table 16 below by referring to the method of Example 1, except that the ratio of the two medicinal materials was adjusted. Then, the decreased percentage of uric acid of these extracts on day 7, day 14, and day 21 of administration (test days) and the mean degree of blood pressure reduction (%) of these extracts were examined in accordance with the methods in Experimental example 1 and Experimental example 2. The results were as shown in Table 16.

Table 16 Uric acid reduction level and blood pressure reduction level

| Sample | Feed ratio of celery seed to sophora flower bud | Decreased percentage of uric acid on day 7 of administration | Decreased percentage of uric acid on day 14 of administration | Decreased percentage of uric acid on day 21 of administration | Mean degree of blood pressure reduction (%) |
|---|---|---|---|---|---|
| Extract 4 | 0: 100 | 95 | 90 | 92 | 7 |
| Extract 5 | 50: 100 | 94 | 89 | 84 | 13 |
| Extract 6 | 100: 100 | 90 | 86 | 81 | 11 |
| Extract 7 | 250: 100 | 71 | 66 | 68 | 12 |
| Extract 8 | 350: 100 | 69 | 66 | 65 | 9 |

(continued)

| Sample | Feed ratio of celery seed to sophora flower bud | Decreased percentage of uric acid on day 7 of administration | Decreased percentage of uric acid on day 14 of administration | Decreased percentage of uric acid on day 21 of administration | Mean degree of blood pressure reduction (%) |
|---|---|---|---|---|---|
| Extract 9 | 500: 100 | 68 | 59 | 65 | 26 |
| Extract 10 | 750: 100 | 70 | 65 | 69 | 37 |
| Extract 11 | 1000: 100 | 73 | 70 | 67 | 42 |

In general, those skilled in the art usually considered the effect of reducing uric acid as satisfactory when the decreased percentage of uric acid was lower than 80%, particularly lower than 75%, while those skilled in the art usually considered the effect as unacceptable when the mean degree of blood pressure reduction was above 15%, particularly above 20%, which was generally considered as an adverse drug reaction.

According to the above results, only the extracts obtained when celery seed : sophora flower bud was in the range of 2 to 4:1 were not only capable of reducing serum uric acid continually, but also would not cause the adverse reaction of lowering blood pressure. However, when the usage amount of celery seed was reduced, the purpose of reducing serum uric acid continually could not be achieved (although the side effect of lowering blood pressure was not significant at this time); and when the usage amount of celery seed was higher than the above range, although the obtained extract was capable of reducing serum uric acid significantly and continually, significant adverse reaction of lowering blood pressure would be caused. Therefore, the extracts obtained when the weight ratio of celery seed to sophora flower bud was 2 to 4:1 were optimal.

Those mentioned above are merely specific embodiments of the present disclosure, however, the protection scope of the present disclosure is not limited thereto, and any variation or alternative which is readily occur to those skilled in the art as within the technical scope disclosed in the present disclosure should be encompassed in the protection scope of the present disclosure. Therefore, the protection scope of the present disclosure should be subject to the protection scope of the claims.

Applicability

The present disclosure provides an extract prepared from a combination of celery seed and sophora flower bud. The extract is not only capable of treating or preventing hyperuricemia effectively and/or may be used for treating or preventing gout, but this combination also enables the adverse side effects exhibited by the combination of the two drugs to be overcome.

**Claims**

1. A celery seed and sophora flower bud extract, wherein the extract is obtained by extracting celery seed and sophora flower bud with an alcohol solvent, wherein the ratio of the celery seed to the sophora flower bud is 2:1 to 4:1 by weight.

2. The extract according to claim 1, wherein the alcohol solvent is selected from C1 to C4 alcohol solvents, preferably methanol, ethanol, isopropanol, and n-butanol, and more preferably ethanol.

3. The extract according to claim 2, wherein the alcohol solvent is an aqueous alcohol solution, and the concentration of the aqueous alcohol solution is 50% to 80% by volume, preferably 50% to 70% by volume.

4. The extract according to any one of claims 1 to 4, wherein the extraction method is an ultrasonic extraction method.

5. The extract according to any one of claims 1 to 5, wherein an extraction time is 30 minutes to 60 minutes.

6. A pharmaceutical composition, comprising the extract according to any one of claims 1 to 5 and one or more pharmaceutically acceptable carriers.

**7.** The extract according to any one of claims 1 to 5 or the pharmaceutical composition according to claim 6, for use for preventing and/or treating hyperuricemia.

**8.** The extract according to any one of claims 1 to 5 or the pharmaceutical composition according to claim 6, for use for preventing and/or treating gout.

**Patentansprüche**

**1.** Extrakt aus Selleriesamen und Sophora-Blütenknospen, wobei der Extrakt durch Extraktion von Selleriesamen und Sophora-Blütenknospen mit einem alkoholischen Lösungsmittel erhalten wird, wobei das Gewichtsverhältnis von Selleriesamen zu Sophora-Blütenknospen 2:1 bis 4:1 beträgt.

**2.** Extrakt nach Anspruch 1, wobei das alkoholische Lösungsmittel ausgewählt ist aus C1- bis C4-Alkohol-Lösungsmitteln, vorzugsweise Methanol, Ethanol, Isopropanol und n-Butanol, und weiter vorzugsweise Ethanol.

**3.** Extrakt nach Anspruch 2, wobei das alkoholische Lösungsmittel eine wässrige Alkohollösung ist und die Konzentration der wässrigen Alkohollösung 50 bis 80 Volumenprozent, vorzugsweise 50 bis 70 Volumenprozent, beträgt.

**4.** Extrakt nach einem beliebigen der Ansprüche 1 bis 4, wobei das Extraktionsverfahren ein Ultraschallextraktionsverfahren ist.

**5.** Extrakt nach einem beliebigen der Ansprüche 1 bis 5, wobei die Extraktionszeit 30 Minuten bis 60 Minuten beträgt.

**6.** Pharmazeutische Zusammensetzung, umfassend den Extrakt nach einem beliebigen der Ansprüche 1 bis 5 und einen oder mehrere pharmazeutisch akzeptable Träger.

**7.** Extrakt nach einem beliebigen der Ansprüche 1 bis 5 oder pharmazeutische Zusammensetzung nach Anspruch 6, zur Verwendung zum Vorbeugen und/oder Behandeln von Hyperurikämie.

**8.** Extrakt nach einem beliebigen der Ansprüche 1 bis 5 oder die pharmazeutische Zusammensetzung nach Anspruch 6, zur Verwendung zum Vorbeugen und/oder Behandeln von Gicht.

**Revendications**

**1.** Extrait de graine de céleri et de bouton floral de Sophora, où l'extrait est obtenu par extraction de graine de céleri et de bouton floral de Sophora avec un solvant alcoolique, où le rapport de la graine de céleri au bouton floral de Sophora est de 2:1 à 4:1 en poids.

**2.** Extrait selon la revendication 1, où le solvant alcoolique est choisi parmi des solvants alcooliques en C1 à C4, de préférence le méthanol, l'éthanol, l'isopropanol et le n-butanol, et plus préférablement l'éthanol.

**3.** Extrait selon la revendication 2, dans lequel le solvant alcoolique est une solution alcoolique aqueuse, et la concentration de la solution alcoolique aqueuse est de 50 % à 80 % en volume, de préférence de 50 % à 70 % en volume.

**4.** Extrait selon l'une quelconque des revendications 1 à 4, dans lequel le procédé d'extraction est un procédé d'extraction par ultrasons.

**5.** Extrait selon l'une quelconque des revendications 1 à 5, dans lequel le temps d'extraction est de 30 minutes à 60 minutes.

**6.** Composition pharmaceutique, comprenant l'extrait selon l'une quelconque des revendications 1 à 5 et un ou plusieurs véhicules pharmaceutiquement acceptables.

**7.** Extrait selon l'une quelconque des revendications 1 à 5 ou composition pharmaceutique selon la revendication 6, pour utilisation pour la prévention et / ou le traitement de l'hyperuricémie.

**8.** Extrait selon l'une quelconque des revendications 1 à 5 ou composition pharmaceutique selon la revendication 6, pour utilisation pour la prévention et/ou le traitement de la goutte.

**FIG. 1**

**FIG. 2**

24

**FIG. 3**

**FIG. 4**

**FIG. 5**

**FIG. 6**

**FIG. 7**

**FIG. 8**

FIG. 9

FIG. 10

FIG. 11

FIG. 12

**FIG. 13**

Day 35

**FIG. 14**

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 101007015 A

**Non-patent literature cited in the description**

- British Herbal Pharmacopoeia. 1983
- **TONG GUO-HUI et al.** Effect of Celery Seed Extract on Hyperuricemia in Rats. *Food Science,* 2008, vol. 29 (6), 641
- **LI LI-YU ; LIN ZHI-JIAN ; ZHANG BING et al.** Effect of high fructose drinking water on uric acid level in rats and the underlying pathological mechanism. *Chinese Journal of Clinical Nutrition,* 2014, vol. 22 (6), 368-374

- **JIN SHEN-RUI ; ZHENG JUN ; LIU SHAO-TANG.** Preliminary study on hyperuricemia model in mice. *Journal of Chengdu University of Traditional Chinese Medicine,* 1999, vol. 22 (01), 50-65
- **JI WEI ; ZHU XUAN-XUAN ; TAN WEN-FENG ; LU YAN.** Effects of Rebixiao granules on blood uric acid in patients with repeatedly attacking acute gouty arthritis. *Chinese Journal of Integrative Medicine,* 2005, vol. 11 (1), 15-21